# EUROPEAN PATENT APPLICATION

(11) **EP 0 696 641 A1**
(43) Date of publication of application: **14.02.1996**
(21) Application number: 94914567.6
(22) Date of filing: 28.04.1994
(51) Int. Cl.: C12Q 1/48

(54) **METHOD OF DETERMINING POTASSIUM IONS**

(30) Priority: 30.04.1993 JP 104154/93
(71) Applicant: Kyowa Medex Co. Ltd., Tokyo 104 (JP)
(72) Inventor: TADANO, Toshio, Numazu-shi, Shizuoka 410 (JP); MIIKE, Akira, Sunto-gun, Shizuoka 411 (JP); UMEMOTO, Jun, Miki-shi, Hyogo 673-07 (JP)
(74) Representative: VOSSIUS & PARTNER
(86) International application number: JP9400714
(87) International publication number: WO9425624

(57) **Abstract**

A method of determining potassium ions contained in a sample by conducting an enzymatic reaction using a pyruvate kinase in an aqueous medium, wherein the enzymatic reaction is conducted in the presence of 80-280 mM lithium ions. This method enables potassium ions to be readily and rapidly determined with good accuracy and reproducibility.

## Description

### Technical Field

This invention relates to a method for quantitatively determining potassium ions using an enzyme reaction in the presence of lithium ions.

### Background Art

As a method for chemically determining the amount of potassium ions in a biosample, there is known a method utilizing an enzyme reaction using a pyruvate kinase which increases its activity in proportion to the amount of potassium ions. According to this method, however, an accurate determination cannot be achieved due to the interference action of sodium ions in a sample.

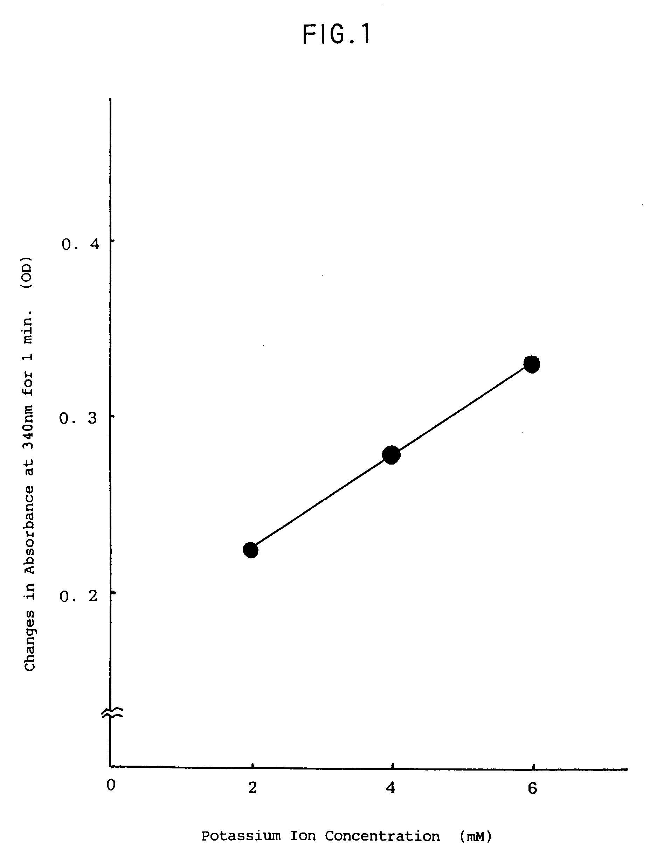

In order to prevent the interference of sodium ions, a method has been developed in which the enzyme reaction is carried out in the presence of lithium ions [Clinical Chemistry, 35:817-820 (1989); Japanese Unexamined Patent Publication/PCT No. 1-503596]. With respect to this method, it is known that when the sodium ion concentration in a sample differs by 10 mM, the measured error of the potassium ion concentration in the sample is at least 0.1 mM [Clinical Chemistry, 35:819 (Fig. 1)(1989)]. Therefore, it is necessary to correct the measured error of the potassium ion concentration which depends on the influence of the coexisting sodium ion concentration. Accordingly, it is also necessary to determnine the coexisting sodium ion concentration in the sample (Japanese Unexamined Patent Publication/PCT No. 1-503596).

The lithium ion can suppress the interference of the sodium ion, but it also can interfere with the potassium ion. Therefore, it has been reported that the sole-use of lithium ions in the determination of potassium ions using a pyruvate kinase does not enable an accurate determination [Koso (Enzyme), Dickson Webb, p. 396, (1970), Hakusui-Sha Co., Ltd.].

Accordingly, in the quantitative determination method for potassium ions utilizing an enzyme reaction using a pyruvate kinase, the sole-use of lithium ions cannot eliminate the error depended on the influence of sodium ions completely, thereby it requires the correcting the error. Since the correcting the error is complicated, a more simple determination method is desired.

On the other hand, there is known an improved method wherein a bicyclic crown ether such as Cryptfix™ 221 is used as a sodium binder together with lithium ions in order to remove sodium ions. However, the dissociation rate of the crown ether is small and a long time is for the prereaction before the start of measurement. Thus, this improved method lacks prompt measuring operation. The development of a superior method is desired.

### Disclosure of the Invention

The present invention has been achieved based on the following our finding. In a pyruvate kinase reaction, the suppressing effect of high concentration lithium ions on sodium ion is very strong, but that on potassium ion is extremely weak. It is the object of the present invention to provide a quantitative determination method for potassium ions using high concentration lithium ions solely, in which the measurement error become very small and the correction is not needed.

In other words, as a method for quantitatively determining potassium ions in a sample utilizing an enzyme reaction using a pyruvate kinase in an aqueous medium, the present invention is characterized by carrying out the enzyme reaction in the presence of lithium ions at a concentration of 80-280 mM.

According to the method of the present invention, it is possible to sufficiently suppress the interference of sodium ions in a sample and thus to determine the amount of potassium ions in the sample accurately.

In the present invention, "an aqueous medium" means a liquid containing water, such as a buffer solution and physiological saline. As examples of the buffer solution, tris(hydroxymethyl) aminomethane-HCl buffer (hereinafter referred to as "Tris-HCl buffer"), phosphate buffer, acetate buffer, succinate buffer, oxalate buffer, phthalate buffer, borate buffer, glycine buffer, 3-(N-morpholino)propanesulfonate (MOPS) buffer, barbital buffer, Good's buffer and the like may be enumerated. These buffer solutions may be used independently or in combination. These buffer solutions are used at a concentration of 5-500 mM at pH 6.5-8.5, preferably 6.5-7.5. When an acidic buffer is used, it is preferable to adjust the pH by using lithium hydroxide or the like. In addition, it is preferable to remove as much as possible the sodium ions, potassium ions, etc. present in an aqueous medium as impurities by means of ion exchange or the like.

As a sample containing potassium ions, any sample may be used as long as it is miscible with an aqueous medium. According to the present invention, biosamples such as blood, serum, urine and cells that are difficult to measure by the atomic absorption spectrometry can be measured.

As a source for lithium ions, lithium chloride, lithium nitrate, lithium sulfate, lithium perchlorate, lithium carbonate, lithium hydrogencarbonate, lithium hydroxide, lithium borohydride and the like may be enumerated. The amounts of these compounds are adjusted to give a lithium ion concentration of 80-280 mM in the reaction solution.

As a pyruvate kinase, an enzyme belonging to the enzyme number EC. 2.7.1.40 which is derived from a higher animal or a microorganism, or an enzyme which is obtained by modifying the above-mentioned enzyme by genetic engineering techniques may be used. Preferably, an enzyme derived from *Bacillus stearothermophilus* is used.

As a substrate and a coenzyme for a pyruvate kinase reaction, phosphoenolpyruvic acid and adenosine diphosphate (ADP) are used. Since divalent manganese works as a cofactor in an enzyme reaction using a pyruvate kinase derived from a microorganism such as *Bacillus stearothermophilus*, it is preferable to add divalent manganese in a microorganism-derived pyruvate kinase reaction. In addition, since divalent magnesium works as a cofactor in an enzyme reaction using a pyruvate kinase derived from a higher animal, such as a pyruvate kinase from rabbit muscle, it is preferable to add divalent magnesium in a higher animal-derived pyruvate kinase reaction.

In the present invention, any method may be used for the activity measurement of a pyruvate kinase in the enzyme reaction. Preferably, a method for determining the amount of pyruvic acid produced by the enzyme reaction is used.

As a method for determining the amount of pyruvic acid, there may be used a method in which pyruvic acid is reacted with a pyruvate oxidase in an aqueous medium and then the amount of the hydrogen peroxide produced therefrom is determined; a method in which changes in absorbance are measured when 2,4-dinitrophenylhydrazine is converted into hydrazone by pyruvic acid; and the like. Preferably, a method is used wherein pyruvic acid is reacted with a lactate dehydrogenase (EC. 1.1.1.27) in the presence of reduced nicotinamide adenine dinucleotide (NADH) and then the amount of decreased NADH or increased nicotinamide adenine dinucleotide (NAD) is measured by absorptiometry, fluorescent photometry or the like.

The lactate dehydrogenase used here may be any lactate dehydrogenase. An enzyme derived from an animal, plant or microorganism, or an enzyme which is obtained by modifying the above enzyme by genetic engineering techniques may be used.

Now, preferred embodiments of the quantitative determination method for potassium ions according to the present invention will be described below.

To an aqueous medium containing lithium ions (at a final concentraton of 80-280 mM in the reaction solution), phosphoenolpyruvic acid (at a final concentraton of 0.3-30 mM in the reaction solution), ADP (at a final concentration of 0.5-10 mM in the reaction solution) and NADH (at a final concentration of 0.1-1 mM in the reaction solution), a sample containing potassium ion is added. The resultant mixture is mixed at 4-50 °C for 30 seconds or more to give a pretreated solution.

An aqueous medium containing a pyruvate kinase (at a final concentration of 200-10000 U/liter in the reaction solution), lactate dehydrogenase (at a final concentration of 5000-100000 U/liter in the reaction solution) and a cofactor for the pyruvate kinase (at a final concentration of 1-100 mM in the reaction solution) is heated to 4-50°C. Then, this medium is added to the above-mentioned pretreated solution and reacted at 4-50°C for 30 seconds or more. At this time, the reaction solution is adjusted to have, preferably, a pH value of 7.5 or below at 25 °C. The amount of decrease in NADH after the reaction is measured by absorptiometry or fluorescent photometry and then the amount of potassium corresponding thereto is calculated.

In the pyruvate kinase reaction, a glutamate dehydrogenase (2500-20000 U/liter) and α-ketoglutaric acid for the removal of ammonia, surfactants such as polyethylene glycol monotrinitrophenylmethyl (Triton X) for the prevention of the occurrence of turbidity, proteins for stabilization such as bovine serum albumin, human serum albumin, ovalbumin, etc., salts for solubilization such as calcium chloride, magnesium chloride, sodium chloride, etc., and glycerol or the like for the preservation and stabilization of reagents may be added to the reaction solution, if necessary.

The method of the present invention using lithium ions does not require a long time for the prereaction before the start of measurement nor damage the stability of glutamate dehydrogenase, lactate dehydrogenase and pyruvate kinase. Therefore, according to the present invention, there is provided a novel method for quantitatively determining potassium ions in a biosample which is good in accurate determination, simple, and excellent in the preservation and stability of reagents.

### Brief Description of the Drawings

Fig. 1 is a graph showing a calibration curve for potassium ions obtained by using 120 mM lithium ions.

Fig. 2 is a graph showing measurement errors in potassium ions against lithium ion concentrations. In this Fig., marks ―●― and ―○― represent the measurement errors in potassium ions when a *Bacillus stearothermophilus*-derived enzyme is used and when a rat cardiac muscle-derived enzyme is used, respectively.

### Best Mode for Carrying Out the Invention

The present invention will be described in more detail with reference to the following Examples, which should not be construed as limiting the scope of the present invention.

### (Example 1)

### (1) Preparation of Standard Solutions for Obtaining Potassium Ion Calibration Curves

Potassium chloride (Wako Pure Chemicals) was diluted with distilled water to prepare standard solutions for obtaining potassium ion calibration curves so that the final concentrations in the reaction solution become 2, 4 and 6 mM. In the resultant standard solutions, sodium chloride (at a concentration of 140 mM in the reaction solution) was allowed to coexist.

### (2) Preparation of Reagents for the Quantitative Determination of Potassium Ions

### (a) The first reagent having the following composition was prepared.

In 100 ml of 300 mM Tris-HCl buffer (25°C, pH 7.8), there were dissolved 80 mg (3.8 mM in the reaction solution) of sodium phosphoenolpyruvate (Boehringer Mannheim), 32 mg (0.45 mM in the reaction solution) of β-NADH (Oriental Yeast) and 160 mg (3.4 mM in the reaction solution) of ADP (Oriental Yeast), and lithium chloride (prepared by ion exchange) was further dissolved therein to give a concentration of 120 mM in the reaction solution.

### (b) The second reagent having the following composition was prepared.

In 50 ml of 10 mM MOPS buffer of which the pH was adjusted at 6.6 by using lithium hydroxide, a *Bacillus stearothermophilus-*derived pyruvate kinase (Unitika Ltd.) (420 U/liter in the reaction solution), 1.2 ml (10000 U/liter in the reaction solution) of lactate dehydrogenase (glycerol solution manufactured by Boehringer Mannheim), 2.0 ml (10 mM in the reaction solution) of 250 mM aqueous solution of manganese chloride and 3 g (660 mM in the reaction solution) of glycerol were dissolved.

### (3) Quantitative Determination of Potassium Ions

0.05 ml of the standard solution for obtaining potassium ion calibration curves prepared in (1) above was placed in a test tube. Then, 2.0 ml of the first reagent was added thereto, mixed and incubated for 3 minutes. To the resultant solution, 1 ml of the second reagent which had been incubated at 37°C in advance was added and mixed. Then, changes in absorbance at 340 nm for 1 minute from 1 to 2 minutes after the mixing were measured. The working curve obtained is shown in Fig. 1.

### (Example 2)

### (1) Dependence on Lithium Ion Concentration in the Case of Using a Bacillus stearothermophilus-Derived Enzyme

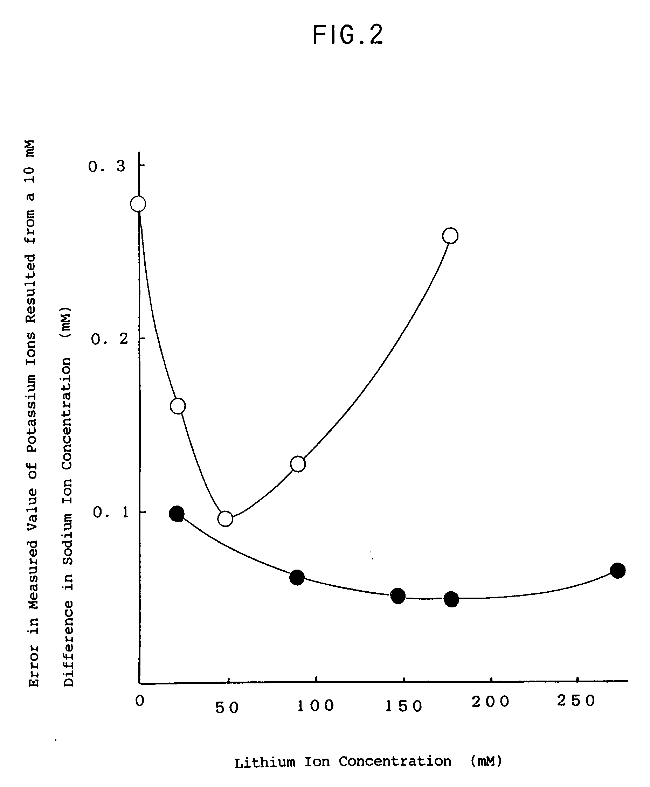

Potassium ions were quantitatively determined in the same manner as in Example 1 except that the first reagent was so prepared to have a lithium chloride concentration of 30-420 mM (20-280 mM in the reaction solution) and to give a sodium chloride concentration of 130, 140 or 150 mM in the reaction solution. Fig. 2 shows the errors in the measured values of potassium ions depended on a 10 mM difference in sodium ion concentration.

### (2) Dependence on Lithium Ion Concentration in the Case of Using a Rat Cardiac Muscle-Derived Enzyme

Potassium ions were quantitatively determined in the same manner as in (1) of Example 2 except that a rat cardiac muscle-derived pyruvate kinase (Boehringer Mannheim) was used instead of a *Bacillus stearothermophilus*-derived pyruvate kinase and that magnesium chloride was used instead of manganese chloride. Fig. 2 shows the errors in the measured values of potassium ions depended on a 10 mM difference in sodium ion concentration.

According to Fig. 2, when a *Bacillus stearothermophilus*-derived enzyme was used, the error was 0.06 mM or less at a lithium ion concentration of 80-280 mM, whereas the error was about 0.1 mM at a lithium ion concentration of 20 mM disclosed in Japanese Unexamined Patent publication/PCT No. 1-503596. When a rat cardiac muscle-derived enzyme was used, the error was 0.1 mM or more at any lithium ion concentration.

### Industrial Applicability

According to the present invention, there is provided a method which is good in accurate determination and reproducibility and enables a simple and quick quantitative determination of potassium ions.

## Claims

1. A method for quantitatively determining potassium ions in a sample utilizing an enzyme reaction using a pyruvate kinase in an aqueous medium, wherein said enzyme reaction is carried out in the presence of lithium ions at a concentration of 80-280 mM.

2. The method according to claim 1, wherein said pyruvate kinase is a *Bacillus stearothermophilus*-derived enzyme.
